(19) Europäisches Patentamt
European Patent Office
Office européen des brevets

(11) **EP 4 202 949 A1**

(12) **EUROPEAN PATENT APPLICATION**

(43) Date of publication:
**28.06.2023 Bulletin 2023/26**

(51) International Patent Classification (IPC):
***G16H 30/40*** *(2018.01)* ***G16H 50/20*** *(2018.01)*

(21) Application number: **22150460.8**

(22) Date of filing: **06.01.2022**

(52) Cooperative Patent Classification (CPC):
**G16H 30/40; G16H 50/20**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.12.2021 US 202163293215 P**

(71) Applicant: **Koninklijke Philips N.V.**
**5656 AG Eindhoven (NL)**

(72) Inventors:
• **CHEN, Alvin**
**Eindhoven (NL)**
• **RAJU, Balasundar Iyyavu**
**Eindhoven (NL)**

(74) Representative: **Philips Intellectual Property & Standards**
**High Tech Campus 52**
**5656 AG Eindhoven (NL)**

(54) **METHODS AND SYSTEMS FOR CLINICAL SCORING OF A LUNG ULTRASOUND**

(57) A method (100) for generating an ultrasound exam score, comprising: (i) receiving (104) a temporal sequence of ultrasound image data for each of a plurality of different lungs zones; (ii) identifying (106) using a feature identification algorithm, for each zone, one or more features of the lung(s); (iii) determining (108), for each identified feature, a confidence score; (iv) determining (110), using a composite score algorithm, a composite score for each zone indicative of a disease or condition severity or a diagnosis; (v) determining (112) a lung score using a lung score algorithm, indicative of a disease or condition severity or a diagnosis for the lung(s); and (vi) providing (114), via a user interface, one or more of the lung score, the composite score for one or more of the plurality of different zones, and the confidence score for one or more of the identified one or more features.

FIG. 1

EP 4 202 949 A1

**Description**

GOVERNMENT INTEREST

This invention was made with United States government support awarded by the United States Department of Health and Human Services under the grant number HHS/ASPR/BARDA 75A50120C00097. The United States has certain rights in this invention.

FIELD OF THE INVENTION

The present disclosure is directed generally to methods and systems for generating an ultrasound exam score.

BACKGROUND

Lung ultrasound imaging is an important tool for disease screening, monitoring, diagnostic support, and management. Important clinical features - such as B-lines, merged B-lines, pleural line changes, consolidations, and pleural effusions, among others - can be identified using lung ultrasound. The combined presence of these features are predictors of a range of pulmonary and infectious diseases, including COVID-19 pneumonia. However, identifying clinical features using lung ultrasound is a challenging skill to learn, and success typically requires extensive specialized training and experience.

During an ultrasound exam, images or video are acquired at multiple lung zones, sometimes as many as fourteen zones for a single exam. These zones are then examined individually, and the results are aggregated to determine an overall diagnosis or severity for the entire exam. Proper interpretation of lung ultrasound features for each video, as well as the overall interpretation at the exam level, requires specialized training and expertise.

Automated quantification tools offer the potential to simplify and standardize image interpretation tasks, including ultrasound analysis. Studies have shown a correlation between automated lung ultrasound features and expert ratings, as well as correlation to gold standard measurements such as blood tests or chest CT. Automated analysis may even be used diagnostically for conditions such as COVID-19 pneumonia. However, automated quantification tools have significant limitations. One limitation of these tools is their black-box nature, making it difficult to understand or interpret the decision-making process of the tools. This results in challenges in clinical adoption and increased burden for regulatory clearance. This lack of transparency is especially problematic in the case of lung ultrasound, since it involves aggregating the results across multiple videos. Lack of interpretability at the video level makes it difficult for a clinician to arrive at a meaningful overall exam-level decision.

SUMMARY OF THE INVENTION

Accordingly, there is a need for automated lung ultrasound quantification tools capable of producing and presenting exam-level information in an understandable and interpretable manner.

The present disclosure is directed to inventive methods and systems for analysis of ultrasound lung imaging. Various embodiments and implementations herein are directed to an ultrasound analysis system comprising an ultrasound device configured to obtain an ultrasound image of the patient's lungs, comprising a temporal sequence of ultrasound image data for each of a plurality of different zones of the patient's lungs. The system identifies, using a feature identification algorithm, for each of the plurality of different zones from the temporal sequence of ultrasound image data, one or more features of the lungs. A confidence score is then determined for each of the identified features. A composite score is determined, using a composite score algorithm and the identified features and determined confidence scores, for each of the plurality of different zones. A lung score is then determined using a lung score algorithm analyzing the plurality of determined composite scores. A user interface is used to provide information to a user, possibly including one or more of the lung score, the composite score for one or more of the plurality of different zones, and the confidence score for one or more of the identified one or more features.

Generally in one aspect, a method for generating an ultrasound exam score is provided. The method includes: (i) receiving a temporal sequence of ultrasound image data for each of a plurality of different zones of one or both lungs of a patient; (ii) identifying using a feature identification algorithm, for each of the plurality of different zones from the temporal sequence of ultrasound image data, one or more features of the lung(s); (iii) determining, for each of the identified one or more features, a confidence score; (iv) determining, using a composite score algorithm analyzing the identified features and determined confidence scores, a composite score for each of the plurality of different zones, wherein the composite score for a zone is indicative of either a disease or condition severity for a zone or a diagnosis for a zone; (v) determining a lung score using a lung score algorithm analyzing the plurality of determined composite scores, wherein the lung score is indicative of either a disease or condition severity for the lung(s) or a diagnosis for the lung(s); and (vi) providing, via a user interface, one or more of the lung score, the composite score for one or more of the plurality of different zones, and the confidence score for one or more of the identified one or more features.

According to an embodiment, the temporal sequence of ultrasound image data comprises a plurality of frames for each of the plurality of zones, and further wherein a confidence score is determined based on an aggregation of a per-frame confidence score determined for each frame of the plurality of frames.

According to an embodiment, identifying one or more features of the lung comprises determining a presence or absence of a feature.

According to an embodiment, the composite score algorithm determines a composite score for a zone by aggregating two or more composite scores for the zone.

According to an embodiment, the method further includes weighting one or more composite scores, wherein the lung score algorithm utilizes the weighted composite score(s) when determining the lung score.

According to an embodiment, the method further includes receiving, via the user interface, a request for additional information about a user-selected lung zone; and providing, via the user interface, the requested additional information about the user-selected lung zone, wherein the additional information comprises one or more of the temporal sequence of ultrasound image data for user-selected lung zone, the composite score for the user-selected lung zone, and the confidence score for the user-selected lung zone.

According to an embodiment, the composite score for each of the plurality of different zones is determined by aggregating the identified features for the respective zone.

According to an embodiment, the composite score for each of the plurality of different zones is determined by aggregating the same identified feature across the plurality of zones.

According to another aspect is a method for generating an ultrasound exam score. The method includes: (i) receiving a temporal sequence of ultrasound image data for each of a plurality of different zones of one or both lungs of a patient; (ii) determining, using a composite score algorithm analyzing the ultrasound image data, a composite score for each of the plurality of different zones, wherein the composite score for a zone is indicative of either: a disease or condition severity for a zone; or a diagnosis for a zone; (iii) determining a lung score using a lung score algorithm analyzing the plurality of determined composite scores, wherein the lung score is indicative of either: a disease or condition severity for the lung(s); or a diagnosis for the lung(s); and (iv) providing, via a user interface, one or more of the lung score, the composite score for one or more of the plurality of different zones, and the confidence score for one or more of the identified one or more features.

According to another aspect is an ultrasound analysis system configured to generate an ultrasound exam score. The system includes: a temporal sequence of ultrasound image data for each of a plurality of different zones of one or both lungs of a patient; a processor configured to: (i) identify using a feature identification algorithm, for each of the plurality of different zones from the temporal sequence of ultrasound image data, one or more features of the lung(s); (ii) determine, for each of the identified one or more features, a confidence score; (iii) determine, using a composite score algorithm analyzing the identified features and determined confidence scores, a composite score for each of the plurality of different zones, wherein the composite score for a zone is indicative of either: (a) a disease or condition severity for a zone; or (b) a diagnosis for a zone; (iv) determine a lung score using a lung score algorithm analyzing the plurality of determined composite scores, wherein the lung score is indicative of either: (a) a disease or condition severity for the lung(s); or (b) a diagnosis for the lung(s); and a user interface configured to provide one or more of the lung score, the composite score for one or more of the plurality of different zones, and the confidence score for one or more of the identified one or more features.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.

Fig. 1 is a flowchart of a method for generating an ultrasound exam score using an ultrasound analysis system, in accordance with an embodiment.
Fig. 2 is a schematic representation of an ultrasound analysis system, in accordance with an embodiment.
Fig. 3 is a schematic representation of composite severity scores derived by the ultrasound analysis system, in accordance with an embodiment.

Fig. 4 is a schematic representation of composite severity scores and lung score reporting by the ultrasound analysis system, in accordance with an embodiment.

Fig. 5 is a flowchart of a method for generating a final lung score using an ultrasound analysis system, in accordance with an embodiment.

Fig. 6 is a flowchart of a method for generating a final lung score using an ultrasound analysis system, in accordance with an embodiment.

Fig. 7A is a flowchart of a method for generating a final lung score using an ultrasound analysis system, in accordance with an embodiment.

Fig. 7B is a flowchart of a method for generating a final lung score using an ultrasound analysis system, in accordance with an embodiment.

Fig. 8 is a flowchart of a method for generating a final lung score using an ultrasound analysis system, in accordance with an embodiment.

Fig. 9 is a flowchart of a method for generating a final lung score using an ultrasound analysis system, in accordance with an embodiment.

Fig. 10 is a flowchart of a method for generating a final lung score using an ultrasound analysis system, in accordance with an embodiment.

Fig. 11 is a schematic representation of lung score reporting by the ultrasound analysis system, in accordance with an embodiment.

## DETAILED DESCRIPTION OF EMBODIMENTS

The present disclosure describes various embodiments of an ultrasound analysis system and method. More generally, Applicant has recognized and appreciated that it would be beneficial to provide an ultrasound analysis that automatically generates a lung exam score or diagnosis. For example, an ultrasound analysis system receives or obtains ultrasound image data for each of a plurality of different zones of the patient's lungs. The system identifies, using a feature identification algorithm, for each of the plurality of different zones from the temporal sequence of ultrasound image data, one or more features of the lungs. A confidence score is then determined for each of the identified features. A composite score is determined, using a composite score algorithm and the identified features and determined confidence scores, for each of the plurality of different zones. A lung score is then determined using a lung score algorithm analyzing the plurality of determined composite scores. A user interface is used to provide information to a user, possibly including one or more of the lung score, the composite score for one or more of the plurality of different zones, and the confidence score for one or more of the identified one or more features.

The ultrasound analysis system and method disclosed or otherwise envisioned herein provides numerous advantages over the prior art. Providing an ultrasound analysis system and method that enables the automated detection and diagnosis of lung disease or a lung condition in an understandable and interpretable manner can prevent serious lung injury, improve patient outcomes, and potentially save lives.

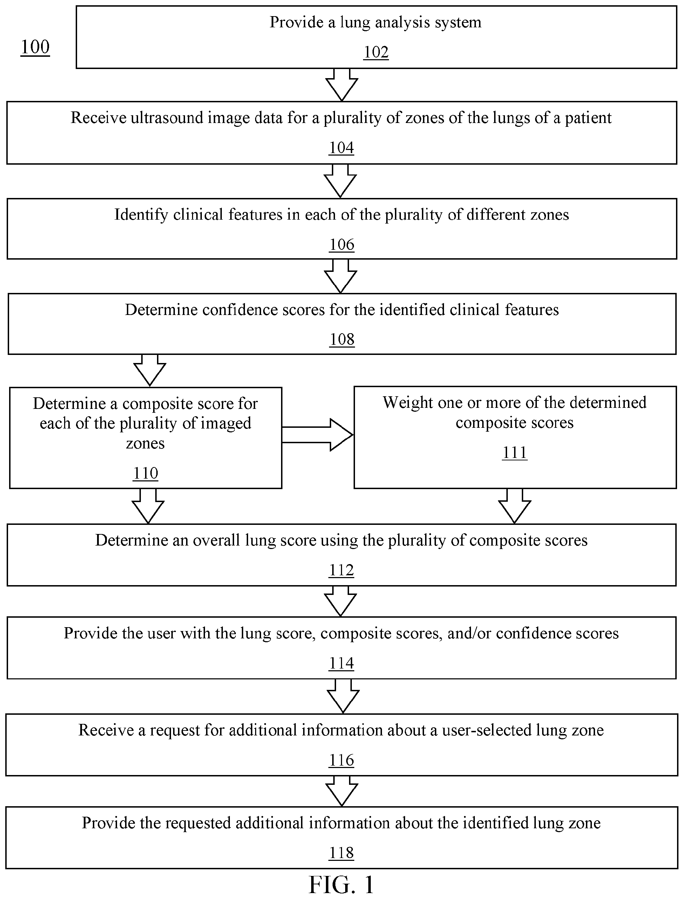

Referring to Fig. 1, in one embodiment, is a flowchart of a method 100 for generating an ultrasound exam score using an ultrasound analysis system. The methods described in connection with the figures are provided as examples only, and shall be understood not to limit the scope of the disclosure. The ultrasound analysis system can be any of the systems described or otherwise envisioned herein. The ultrasound analysis system can be a single system or multiple different systems.

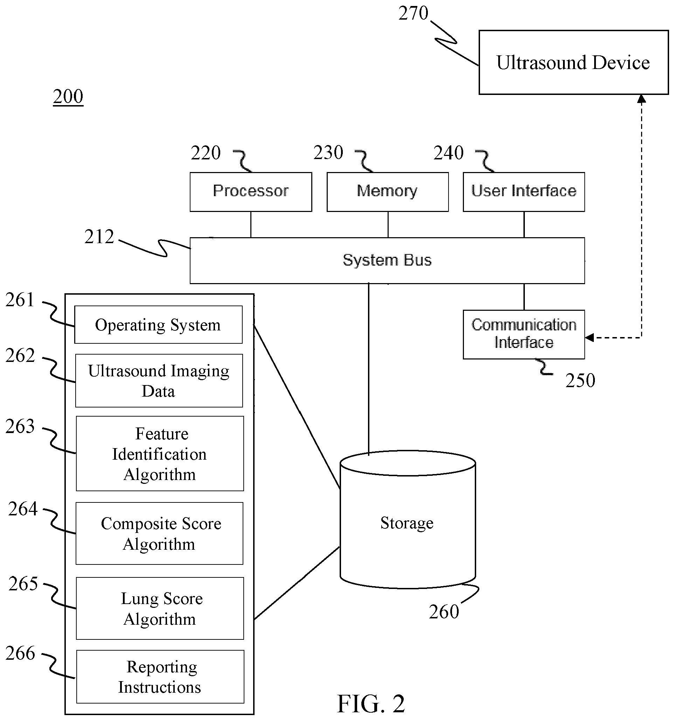

At step 102 of the method, an ultrasound analysis system 200 is provided. Referring to an embodiment of an ultrasound analysis system 200 as depicted in Fig. 2, for example, the system comprises one or more of a processor 220, memory 230, user interface 240, communications interface 250, storage 260, and ultrasound device 270, interconnected via one or more system buses 212. It will be understood that Fig. 2 constitutes, in some respects, an abstraction and that the actual organization of the components of the system 200 may be different and more complex than illustrated. Additionally, ultrasound analysis system 200 can be any of the systems described or otherwise envisioned herein. Other elements and components of system 200 are disclosed and/or envisioned elsewhere herein.

At step 104 of the method, ultrasound image data is sent to, obtained by, or otherwise received by the system. The ultrasound image data may comprise, for example, a temporal sequence of ultrasound image data such as a video comprising a plurality of frames. Ultrasound image data may be obtained for a single region or zone of a patient's lung, or may be obtained for a plurality of different zones. For example, ultrasound image data may be obtained for one, two, or more zones. Some exams may comprise as many as fourteen zones or more, although more or fewer zones are possible. The ultrasound image data may be received by the system in real-time, or may be stored in local and/or remote memory and received by the system at a future point.

The ultrasound image data may be obtained using any ultrasound device or system, which may be any device or system suitable to obtain or otherwise receive ultrasound image data of the patient. One or more parameters of the ultrasound device can be set, adjusted, preprogrammed, or otherwise determined by a healthcare professional. The ultrasound device or system may be remote to, local to, or a component of, the ultrasound analysis system 200.

At step 106 of the method, the ultrasound analysis system identifies one or more features, also called clinical features, of the lungs using the ultrasound image data. According to an embodiment, a feature is any recognizable aspect of a lung. A feature may be a normal aspect of a lung or an abnormal aspect. A feature may be indicative of a healthy lung or a diseased or injured lung, or a lung experiencing any type of lung-related condition. Thus, a feature may be, for example, anything that can be identified within or from ultrasound image data. Examples of features include A-lines, B-lines, merged B-lines, pleural line abnormalities, consolidation, pleural effusion, and many others.

According to an embodiment, the ultrasound analysis system identifies one or more features of the lungs from the ultrasound image data using a feature identification algorithm. The feature identification algorithm can be any algorithm capable of identifying a feature from ultrasound image data The feature identification algorithm may be trained, for example, using a set of feature training data. The feature identification algorithm may comprise a single algorithm configured to or trained to identify every type of feature, or the feature identification algorithm may comprise different algorithms or aspects each configured to or trained to identify a specific feature. According to an embodiment, the feature identification algorithm utilizes a feature detection or segmentation algorithm to localize a feature in each frame, such as by drawing bounding boxes. Each detected feature, such as each bounding box, may comprise a prediction confidence score, as described herein.

According to one embodiment, the ultrasound analysis system identifies features of the lungs from the ultrasound image data on a presence or absence basis using the feature identification algorithm. The system may identify features on a frame-by-frame basis.

According to one embodiment, the ultrasound analysis system identifies features of the lungs from the ultrasound image data across a single video for a lung zone using the feature identification algorithm. This could be, for example, on a presence or absence basis. As just one example, the feature identification algorithm could identify features using only video rather than individual frames. For example, the system could comprise a deep neural network model that takes a full video clip as input, and outputs the identification of features. As another example, the feature identification algorithm could identify features for one on or more frames in a video, and can aggregate identified features across multiple frames.

According to one embodiment, the ultrasound analysis system identifies features of the lungs from the ultrasound image data for each imaged zone of a plurality of different imaged zones. The system may identify features in each zone sequentially, or the system may identify features in two or more zones simultaneously.

At step 108 of the method, the ultrasound analysis system determines a confidence score for each of the identified one or more features. For example, where the ultrasound analysis system identifies features of the lungs from the ultrasound image data on a presence or absence basis using the feature identification algorithm, the identification may comprise a confidence score between 0 and 1.

According to an embodiment where the ultrasound analysis system identifies features of the lungs from the ultrasound image data across a single video for a lung zone using the feature identification algorithm, the algorithm could use the full video clip as input, and output a confidence score for each feature. As another example, the feature identification algorithm could identify features and confidence scores for one or more frames in a video, and can aggregate identified features and confidence scores across multiple frames. For example, for each feature, the system can calculate a total video-level confidence score based on the sum or average of the per-frame confidence scores. Alternatively, the system can use the size of the feature instead of the confidence score. Alternatively, the system can use a combination of these embodiments to identify features, such as (size of feature) x (confidence score of feature) or any other method or equation for aggregation or combination. The system may determine a confidence score for the identified features sequentially, or the system may determine confidence scores for two or more identified features simultaneously.

At step 110 of the method, the ultrasound analysis system determines one or more composite scores for each imaged zone. According to an embodiment, the system comprises a composite score algorithm that utilizes as input one or more of the identified features and determined confidence scores. The determined one or more composite scores for a zone may comprise information about, or otherwise be indicative of, a disease or condition severity for the zone. In addition or alternatively, the determined composite score for a zone may comprise information about, or otherwise be indicative of, a diagnosis of a disease or condition for the zone. The determined composite score for a zone may comprise other information about the zone.

According to an embodiment, the ultrasound analysis system determines a plurality of composite scores for an imaged zone. For example, the composite score algorithm may utilize input for a given zone to generate a first composite score for B-lines indicative of B-line severity or diagnosis, a second composite score for pleural lines, and so on.

Thus, according to an embodiment, the composite score algorithm derives a composite severity score and/or diagnosis for each frame or video for each zone. This may be a rules-based algorithm or a predictive model, such as a machine learning-based algorithm. The composite score could be calculated for each individual frame and then aggregated, or could be calculated directly for a video. Typically, one video is acquired for each scanned lung zone, which would result in a distinct severity score per lung zone.

According to an embodiment, the output of the composite score algorithm may comprise a binary yes/no, a continuous measurement between 0 and 1, an integer severity scale (such as 0-3), or any other informative or useful output. The output could also be a triage recommendation or outcome prediction.

According to an embodiment, the composite score can be based on a combined panel of two or more lung ultrasound features. One example of a panel of features could be A-lines, B-lines, merged B-lines, pleural line abnormalities, consolidation, and pleural effusion. Other panels with more, fewer, and/or other features are possible.

According to an embodiment, the composite score may be derived using a rules-based algorithm. The rules may be obtained from or derived from established guidelines or international consensus recommendations. In the case of lung ultrasound for respiratory and infectious disease management, such guidelines have been reported in the literature. An advantage of this approach is that the algorithm is already validated and accepted within the broader community.

According to an embodiment, a composite score predictive model could be used instead of the rules-based algorithm. This would comprise a machine learning-based approach such as a decision tree, support vector machine, or neural network, among other possibilities. One advantage is that the model could be optimized for the specific diagnosis or severity assessment task.

According to an embodiment, the composite score is derived directly from the raw ultrasound video data for a zone. This could be achieved, for example, by feeding an entire video clip to the system and composite score algorithm, and predicting the overall severity as a single number. Since one video is typically acquired for each scanned lung zone, this would result in a distinct severity score per lung zone. According to an embodiment, the composite score could be calculated on a frame-by-frame basis and then aggregated.

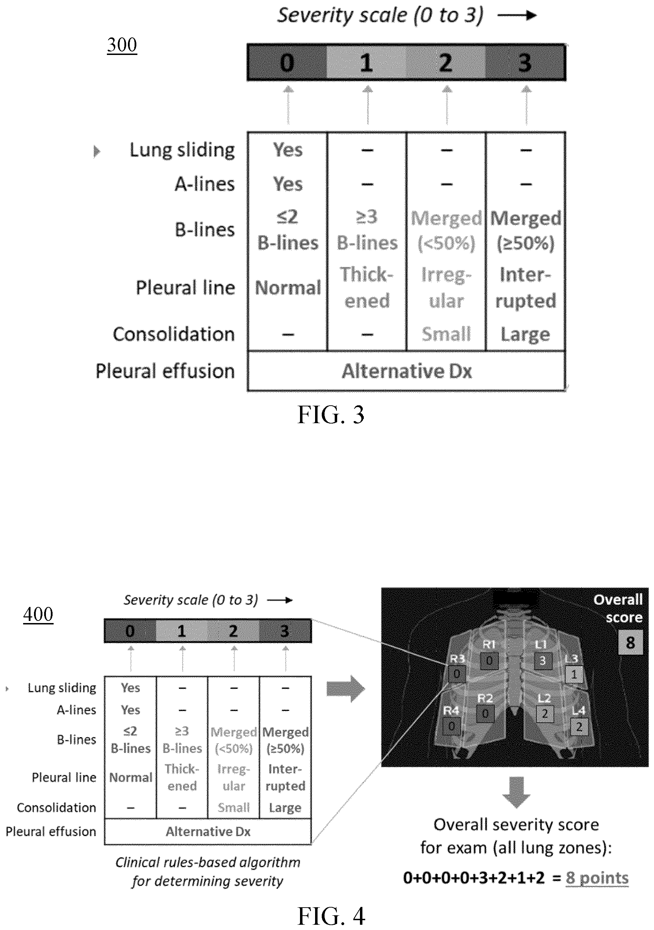

Referring to Fig. 3, in one embodiment, is a summary of possible composite severity scores that can be derived by the composite score algorithm for a zone, using a rules-based algorithm and a scale of 0 to 3, with 0 being least severe (or possibly healthy) and 3 being most severe. For example, a video-level severity score based on a scale between 0 to 3 can be determined from a combination of features present in the video.

As shown in Fig. 3, the ultrasound analysis system is configured to analyze a plurality of features, including but not limited to lung sliding, A-lines, B-lines, pleural line, and consolidation. For each feature, and for each zone, the composite score algorithm utilizes the identified feature and/or feature confidence score to determine a composite score. For example, for the B-lines feature, two or fewer B-lines may result in a severity score of 0, while three or more B-lines may result in a severity score of 1, while less than 50% merged B-lines may result in a severity score of 2, while 50% or more merged B-lines may result in a severity score of 3. Fig. 3 represents only a single possible embodiment of the composite score algorithm analysis and resulting composite score. Many other embodiments are possible.

At step 112 of the method, the ultrasound analysis system determines an overall lung score. According to an embodiment, the system comprises a lung score algorithm that utilizes as input the plurality of determined composite scores for the plurality of imaged lung zones. The determined lung score may comprise information about, or otherwise be indicative of, a disease or condition severity for the patient's lungs. In addition or alternatively, the determined lung score may comprise information about, or otherwise be indicative of, a diagnosis of a disease or condition for the patient's lungs. In addition or alternatively, the determined lung score may comprise other information about the lung.

An ultrasound exam typically comprises a plurality of imaged zones, including as many as fourteen zones or more, although more or fewer zones are possible. The lung score could be the sum or average of the composite scores that were determined for each lung zone. However, other ways of deriving an overall exam-level lung score are possible, and a few of those embodiments are described or otherwise envisioned herein.

Referring to Fig. 4, in one embodiment, is an example of an overall lung score - in this example a severity score - generated from a plurality of composite scores. Ultrasound imaging was analyzed by the ultrasound analysis system to identify features in a plurality of zones (i.e., eight zones being R1-R4 and L1-L4) using a feature identification algorithm. A composite score algorithm was then utilized to determine a composite score for each of the plurality of different zones, as shown in TABLE 1. A final lung score was determined using a lung score algorithm analyzing the plurality of determined composite scores. In this example, the composite scores for the eight imaged zones were summed to generate a lung score of 8 (0+0+0+0+3+2+1+2 = 8). As shown in Fig. 4 and as described further herein, the composite scores and overall score are presented to a user via a diagram comprising the lungs with corresponding composite scores (e.g., "R1" with composite score "0") and the overall score ("Overall score 8").

TABLE 1. Determined Composite Score

| Zone | Composite Score |
|------|-----------------|
| R1 | 0 |
| R2 | 0 |
| R3 | 0 |

(continued)

| Zone | Composite Score |
|------|-----------------|
| R4 | 0 |
| L1 | 3 |
| L2 | 2 |
| L3 | 1 |
| L4 | 2 |

According to an embodiment, the lung score algorithm is configured or trained to determine a per-feature score for each lung zone, aggregate the scores for all zones, and then combine the features at the exam level.

According to an embodiment, some lung zones may be more important than others. To account for this, the overall score could be weighted based on the relative importance of each lung zone. Accordingly, at optional step 111 of the method, the ultrasound analysis system weights one or more of the determined composite scores based on the importance of the corresponding lung zone. The importance of a lung zone may be predetermined by a user of the system, or may be flagged or otherwise indicated during or after a lung exam. At step 112 of the method, the lung score algorithm utilizes the one or more weighted composite scores when determining the lung score. The lung score algorithm could increase the final lung score when summing or otherwise combining or analyzing the composite scores, as a result of utilizing the one or more weighted composite scores. As an example, if L2 is a particularly important zone for or in the exam, any composite score generated for L2 may be weighted. If the composite score for L2 is 0, then the score may not be weighted. Alternatively, if the composite score for L2 is greater than 0, indicating a possible issue, then the composite score can be automatically weighted to reflect the predetermined importance of the L2 zone. As another example, if B-line abnormalities are particularly concerning or important in an exam, a composite score greater than 0 due to B-line abnormalities could be weighted by the system such that it will increase or otherwise impact or affect the determined overall lung score.

According to an embodiment, the lung score could also be determined based on multiple exams on the same patient. This might occur if the patient had exams at different time points during the hospital stay. For example, the ultrasound imaging data for two or more exams may be stored in a database, and can be accessed or otherwise received by the system to perform an analysis per the methods described or otherwise envisioned herein. A lung score may be calculated for each exam, and then may be combined or otherwise aggregated. Differences in the lung score, such as an increase or decrease in the score, may be reported to the user as well in order to show a change over time.

According to an embodiment, if the overall diagnosis or severity assessment requires additional non-ultrasound information, this could be provided as secondary inputs into the final algorithm or model.

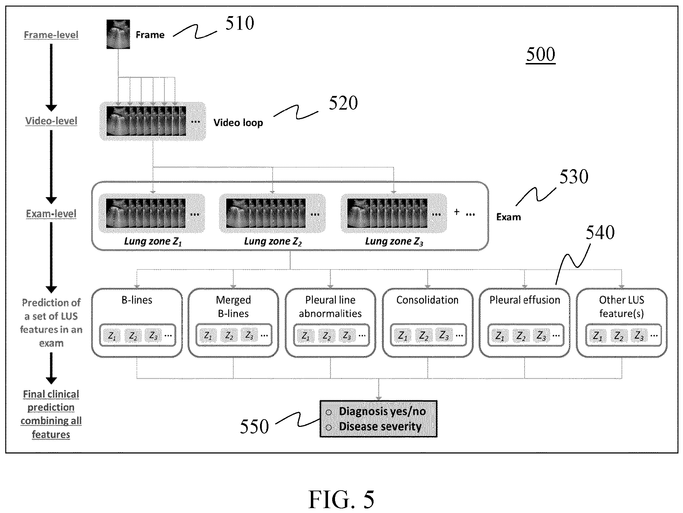

Referring to Fig. 5, in one embodiment, is a flowchart of a method 500 for generating a final lung score using an ultrasound analysis system, which is an embodiment of method 100. According to this embodiment, features are identified or determined at the frame 510, video 520, or exam 530 level to generate a prediction of a set of lung ultrasound (LUS) features 540 in the exam, such as B-lines, merged B-lines, and so on. The set of features generated from the frames, the videos, and/or the exam are then utilized in downstream steps of the method.

According to an embodiment, the data from the generated or extracted features is combined to generate a final lung score 550, which is indicative of disease and/or condition severity, and/or a condition or disease diagnosis.

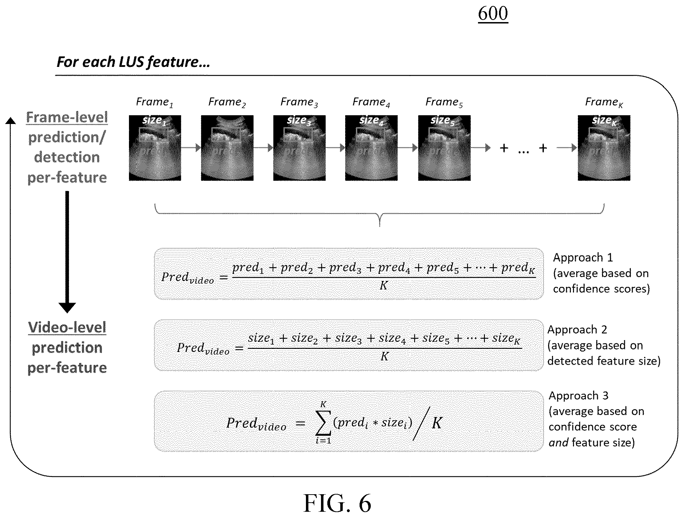

Referring to Fig. 6, in one embodiment, is a flowchart of a method 600 for generating a final lung score using an ultrasound analysis system, which is an embodiment of method 100. According to this embodiment, features are identified or determined at the frame level. In other words, features are identified by the system in each of a plurality of different frames individually for a single zone, and can be repeated for subsequent zones. To generate a video-level identification of or prediction for features, the system can combine frame-level feature determinations in a variety of different ways, including averaging schemes based on confidence scores or feature detection bounding box sizes. For example, using Approach 1, the system can average the confidence scores ($pred_K$) that were determined for the different features ($pred_1 + pred_2 + pred_3 + pred_4 + pred_5... pred_K$). Using Approach 2, the system can average the sizes ($size_K$) that were determined for the different features ($size_1 + size_2 + size_3 + size_4 + size_5... size_K$). Using Approach 3, the system can generate a final lung score from both confidence scores and feature size, using for example the following equation:

$$Pred_{video} = \sum_{i=1}^{K}(pred_i * size_i)/ K \qquad\qquad (Eq. 1)$$

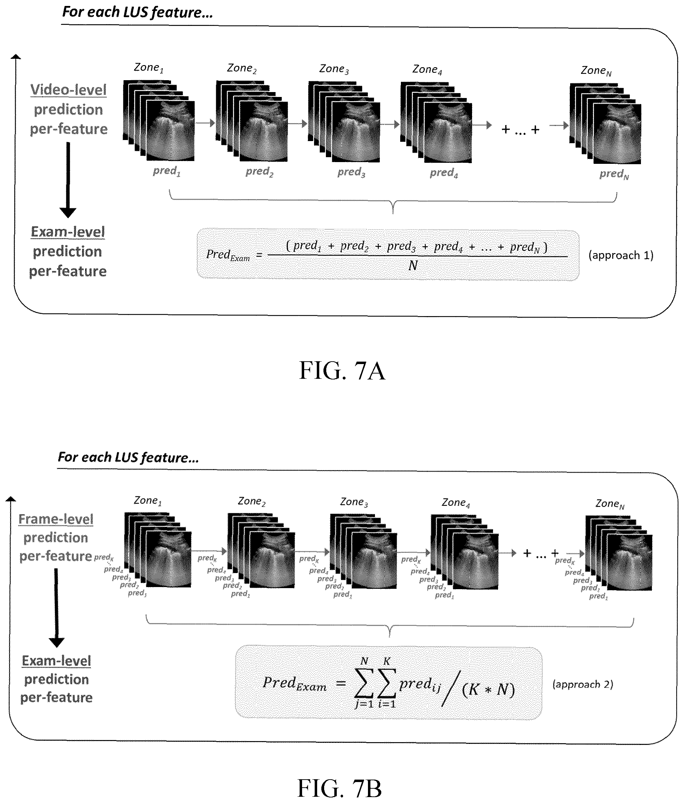

Referring to Fig. 7A, in one embodiment, is a flowchart of a method for generating a final lung score using an ultrasound analysis system, which is an embodiment of method 100. According to this embodiment, features are identified or determined at the video level. Thus, for each zone ($Zone_1$, etc.), a feature prediction ($pred_1$) is made, and the predictions are averaged to generate an exam level prediction using the following equation:

$$Pred_{Exam} = \frac{(pred_1 + pred_2 + pred_3 + pred_4 + pred_5 + pred_n)}{N} \qquad \text{(Eq. 2)}$$

Referring to Fig. 7B, in one embodiment, is a flowchart of a method for generating a final lung score using an ultrasound analysis system, which is an embodiment of method 100. According to this embodiment, features are identified or determined at the frame level. Thus, for each zone ($Zone_1$, etc.), a feature prediction ($pred_1$, etc.) is made for each frame, and the predictions are combined to generate an exam level prediction using the following equation:

$$Pred_{Exam} = \sum_{j=i}^{N} \sum_{i=1}^{K} pred_{ij} / (K * N) \qquad \text{(Eq. 3)}$$

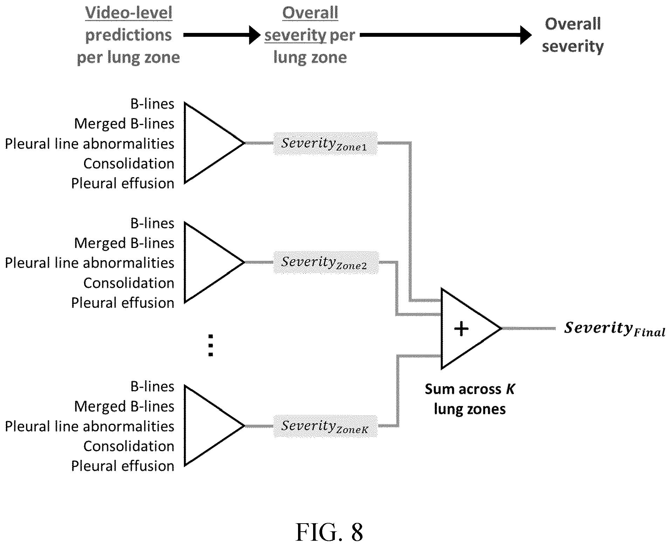

Referring to Fig. 8, in one embodiment, is a flowchart of a method for generating a lung score ($Severity_{Final}$) using an ultrasound analysis system, which is an embodiment of method 100. According to this embodiment, features are identified or determined at the video level. Video-level predictions for each feature are combined to produce an overall severity score for that video for that lung zone ($Severity_{ZoneK}$). The overall severity for the entire exam ($Severity_{Final}$) is derived from the per-video (per lung zone) scores. This could be, for example, a sum of the severity of the plurality of lung zones. The output ($Severity_{Final}$) could be a severity or could be a diagnosis (such as a binary yes/no) instead of or in addition to an overall severity.

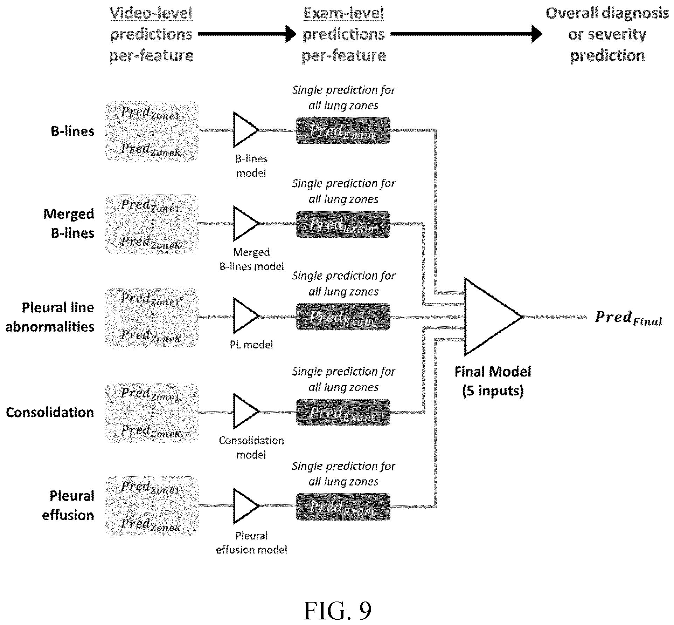

Referring to Fig. 9, in one embodiment, is a flowchart of a method for generating a lung score ($Pred_{Final}$) using an ultrasound analysis system, which is an embodiment of method 100. According to this embodiment, features are identified or determined at the video level for each feature for each zone ($Pred_{Zone1}$... $Pred_{ZoneK}$ for B-lines, $Pred_{Zone1}$ ... $Pred_{ZoneK}$ for Pleural line abnormalities, etc.), and used to generate a confidence score. Thus, video-level predictions for each feature are aggregated across all lung zones to produce exam level predictions per feature across all zones ($Pred_{Exam}$ for B-lines, $Pred_{Exam}$ for Pleural line abnormalities, etc.), which could comprise summing the predictions among other aggregation methods. The exam level feature predictions are fed to the final model. For example, if there are five features, the final model receives and utilizes five inputs. The output ($Pred_{Final}$) can be an overall severity and/or a diagnosis (such as a binary yes/no).

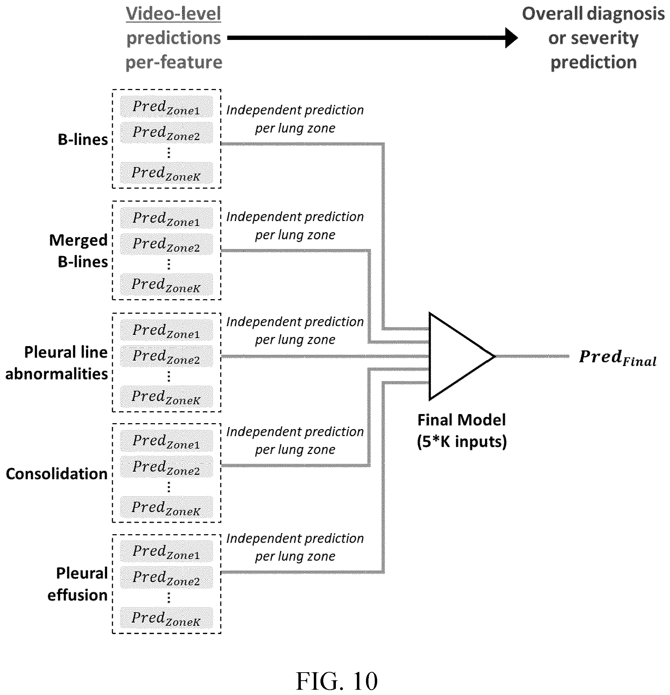

Referring to Fig. 10, in one embodiment, is a flowchart of a method for generating a lung score ($Pred_{Final}$) using an ultrasound analysis system, which is an embodiment of method 100. According to this embodiment, features are identified or determined at the video level for each feature for each zone $Pred_{Zone1}$ ... $Pred_{ZoneK}$ for B-lines, $Pred_{Zone1}$.. $Pred_{ZoneK}$ for Pleural line abnormalities, etc.). The zone predictions are then fed directly into a final model or algorithm to generate the lung score. For example, if there are five features analyzed, the final model utilizes 5*K inputs to generate the lung score.

Returning to method 100 depicted in Fig. 1, at step 114 of the method, the ultrasound analysis system provides information from the analysis to a user via a user interface. The information may comprise one or more of the determined lung score, the composite score for one or more features for one or more of the imaged lung zones, and/or the confidence score for one or more of the identified features. Other information is possible as well, including but not limited to the identity of the patient, patient demographics, diagnosis or treatment information, and a wide variety of other possible information. The information can be provided via the user interface using any method for conveying or displaying information, and the user interface can be any device, interface, or mechanism for providing the conveyed or displayed information.

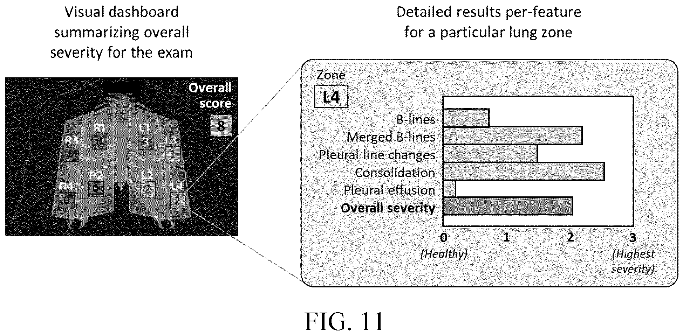

Referring to Fig. 11, in one embodiment, is an example of information provided to a user via a user interface. The information comprises a visual dashboard that displays the composite score for each lung zone ("L1" is "3", etc.), along with the overall exam score ("Overall score" is "8"). The user can click on each lung zone to review more detailed information, including the frame/video level detections for each feature within that lung zone. Similarly, clicking on a lung zone could play the lung ultrasound video acquired at that lung zone, along with the individually detected features.

According to an embodiment, the user interface could summarize information over multiple exams (i.e., multiple time points). One implementation would present a summary dashboard for each time point. A second implementation would plot the overall exam-level score for each time point on a single graph, such as disease progression plot charting the lung severity scores over time.

Accordingly, the methods and systems described or otherwise envisioned herein provide numerous advantages over the prior art. For example, the system provides improved interpretability of ultrasound imaging compared to prior art systems, as a clinician is better able to evaluate the individual features that provide a final lung score. Critically, detecting and visualizing relevant lung features, and providing intermediate results at the frame/video/exam level, allows the user to interpret the ultrasound findings alongside other patient medical information and make a final, more-informed judgment, thereby improving patient outcomes.

According to an embodiment, the methods and systems described or otherwise envisioned herein comprise numerous applications. For example, the system could be utilized in a pre-hospital setting, as an initial evaluation in an emergency room, for follow-up after a treatment, and in many other settings. The method is applicable to all ultrasound imaging systems, especially in point-of-care applications. The methods and systems can be used in a variety of settings including ambulance, ER, or critical care, or surgery situations, including for emergency cases of acute respiratory diseases or thoracic diseases.

At step 116 of the method, the ultrasound analysis system receives, via the user interface, a request for additional information about one or more lung zones. For example, at step 114 of the method information about one or more of the determined lung score, the composite score for one or more features for one or more of the imaged lung zones, and/or the confidence score for one or more of the identified features is displayed on the user interface. At step 116, the user may identify one or more lung zones for expanded information using the user interface. For example, the user may click or touch or otherwise select a lung zone, composite score, or other aspect of the display to indicate the request for additional information.

Referring to Fig. 11, for example, the user has clicked on, touched, or otherwise selected zone L4 for additional information. The user interface interprets the selection as a request for more information about L4, the user-selected lung zone.

At step 118 of the method, the ultrasound analysis system provides, in response to the received request for additional information about one or more lung zones, some or all of the requested information via the user interface. According to an embodiment, the additional information comprises one or more of the temporal sequence of ultrasound image data for user-selected lung zone, the composite score for the user-selected lung zone, the confidence score for the user-selected lung zone, and/or other information about the lung zone.

Referring again to Fig. 11, for example, the user has clicked on, touched, or otherwise selected zone L4 for additional information. The ultrasound analysis system provides additional information to the user via the user interface, shown by the expansion panel on the right-hand side of the figure. The additional information comprises frame/video level detections for each feature within that lung zone, such as the B-lines prediction or composite score (somewhere between 1 and 2), and so on. The prediction or composite score can be an integer or a finer breakdown.

Referring to Fig. 2 is a schematic representation of a ultrasound analysis system 200. System 200 may be any of the systems described or otherwise envisioned herein, and may comprise any of the components described or otherwise envisioned herein. It will be understood that Fig. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may be located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, storage 260 of system 200 may store one or more algorithms, modules, and/or instructions to carry out one or more functions or steps of the methods described or otherwise envisioned herein. For example, the system may comprise, among other instructions or data, ultrasound imaging data 262, feature identification algorithm 263, composite score algorithm 264, lung score algorithm 265, and/or reporting instructions 266, among many other possible instructions and/or data.

According to an embodiment, ultrasound imaging data 262 is any ultrasound imaging data that is sent to, obtained by, or otherwise received by the system. The ultrasound image data may comprise, for example, a temporal sequence of ultrasound image data such as a video comprising a plurality of frames. Ultrasound image data may be obtained for a single region or zone of a patient's lung, or may be obtained for a plurality of different zones. For example, ultrasound image data may be obtained for one, two, or more zones. Some exams may comprise as many as fourteen zones or more, although more or fewer zones are possible. The ultrasound image data may be received by the system in real-time, or may be stored in local and/or remote memory and received by the system at a future point. The ultrasound image data may be obtained using any ultrasound device or system, which may be any device or system suitable to obtain or otherwise receive ultrasound image data of the patient. The ultrasound device or system may be remote to, local to, or a component of, the ultrasound analysis system 200.

According to an embodiment, feature identification algorithm 263 is any model or algorithm that is trained or configured to identify features of the lungs using the ultrasound image data, wherein a feature is anything that can be identified within or from ultrasound image data. Examples of features include A-lines, B-lines, merged B-lines, pleural line abnormalities, consolidation, pleural effusion, and many others. According to an embodiment, the feature identification algorithm 263 is trained or configured to identify features from individual frames or from a video.

According to an embodiment, composite score algorithm 264 is any model or algorithm that is trained or configured to determine a composite score for an imaged zone, utilizing as input one or more of the identified features and determined confidence scores. The determined composite score for a zone may comprise information about, or otherwise be indicative of, a disease or condition severity for the zone. In addition or alternatively, the determined composite score for a zone may comprise information about, or otherwise be indicative of, a diagnosis of a disease or condition for the zone. The determined composite score for a zone may comprise other information about the zone. According to an embodiment, the composite score algorithm derives a composite severity score and/or diagnosis for each frame or video for each zone. This may be a rules-based algorithm or a predictive model, such as a machine learning-based algorithm. According to an embodiment, the output of the composite score algorithm may comprise a binary yes/no, a continuous measurement between 0 and 1, an integer severity scale (such as 0-3), or any other informative or useful output. The output could also be a triage recommendation or outcome prediction.

According to an embodiment, lung score algorithm 265 is any model or algorithm that is trained or configured to determine an overall lung score, utilizing as input the plurality of determined composite scores for the plurality of imaged lung zones. The determined lung score may comprise information about, or otherwise be indicative of, a disease or condition severity for the patient's lungs. In addition or alternatively, the determined lung score may comprise information about, or otherwise be indicative of, a diagnosis of a disease or condition for the patient's lungs. In addition or alternatively, the determined lung score may comprise other information about the lung. An ultrasound exam typically comprises a plurality of imaged zones, including as many as fourteen zones or more, although more or fewer zones are possible. The lung score could be the sum or average of the composite scores that were determined for each lung zone. However, other ways of deriving an overall exam-level lung score are possible, and a few of those embodiments are described or otherwise envisioned herein.

According to an embodiment, reporting instructions 265 direct the system to generate and provide a report or visualization to a user via the user interface 240 of the ultrasound analysis system 200. The report or visualization comprises, for example, information about one or more of the determined lung score, the composite score for one or more features for one or more of the imaged lung zones, and/or the confidence score for one or more of the identified features. Other information is possible as well, including but not limited to the identity of the patient, patient demographics, diagnosis or treatment information, and a wide variety of other possible information. The information can be provided via the user interface using any method for conveying or displaying information, and the user interface can be any device, interface, or mechanism for providing the conveyed or displayed information. According to an embodiment, the instructions may direct the system to display the information on the user interface or display of the system. The report may be communicated by wired and/or wireless communication to another device. For example, the system may communicate the report to a mobile phone, computer, laptop, wearable device, and/or any other device configured to allow display and/or other communication of the report.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

**Claims**

1. A method (100) for generating an ultrasound exam score, comprising:

    receiving (104) a temporal sequence of ultrasound image data for each of a plurality of different zones of one or both lungs of a patient;
    identifying (106) using a feature identification algorithm, for each of the plurality of different zones from the temporal sequence of ultrasound image data, one or more features of the lung(s);
    determining (108), for each of the identified one or more features, a confidence score;
    determining (110), using a composite score algorithm analyzing the identified features and determined confidence scores, a composite score for each of the plurality of different zones, wherein the composite score for a zone is indicative of either: (i) a disease or condition severity for a zone; or (ii) a diagnosis for a zone;
    determining (112) a lung score using a lung score algorithm analyzing the plurality of determined composite scores, wherein the lung score is indicative of either: (i) a disease or condition severity for the lung(s); or (ii) a diagnosis for the lung(s); and
    providing (114), via a user interface, one or more of the lung score, the composite score for one or more of the plurality of different zones, and the confidence score for one or more of the identified one or more features.

2. The method of claim 1, wherein the temporal sequence of ultrasound image data comprises a plurality of frames for each of the plurality of zones, and further wherein a confidence score is determined based on an aggregation of a per-frame confidence score determined for each frame of the plurality of frames.

3. The method of claim 1, wherein identifying one or more features of the lung comprises determining a presence or absence of a feature.

4. The method of claim 1, wherein the composite score algorithm determines a composite score for a zone by aggregating two or more composite scores for the zone.

5. The method of claim 1, further comprising the step of weighting (111) one or more composite scores, wherein the lung score algorithm utilizes the weighted composite score(s) when determining the lung score.

6. The method of claim 1, further comprising the steps of:

    receiving (114), via the user interface, a request for additional information about a user-selected lung zone; and
    providing (116), via the user interface, the requested additional information about the user-selected lung zone, wherein the additional information comprises one or more of the temporal sequence of ultrasound image data for user-selected lung zone, the composite score for the user-selected lung zone, and the confidence score for the user-selected lung zone.

7. The method of claim 1, wherein the composite score for each of the plurality of different zones is determined by aggregating the identified features for the respective zone.

8. A method (100) for generating an ultrasound exam score, comprising:

    receiving (104) a temporal sequence of ultrasound image data for each of a plurality of different zones of one or both lungs of a patient;
    determining (110), using a composite score algorithm analyzing the ultrasound image data, a composite score for each of the plurality of different zones, wherein the composite score for a zone is indicative of either: (i) a disease or condition severity for a zone; or (ii) a diagnosis for a zone;
    determining (112) a lung score using a lung score algorithm analyzing the plurality of determined composite scores, wherein the lung score is indicative of either: (i) a disease or condition severity for the lung(s); or (ii) a diagnosis for the lung(s); and
    providing (114), via a user interface, one or more of the lung score, the composite score for one or more of the plurality of different zones, and the confidence score for one or more of the identified one or more features.

9. An ultrasound analysis system (200) configured to generate an ultrasound exam score, comprising:

    a temporal sequence of ultrasound image data (262) for each of a plurality of different zones of one or both lungs of a patient;

a processor (220) configured to: (i) identify using a feature identification algorithm, for each of the plurality of different zones from the temporal sequence of ultrasound image data, one or more features of the lung(s); (ii) determine, for each of the identified one or more features, a confidence score; (iii) determine, using a composite score algorithm analyzing the identified features and determined confidence scores, a composite score for each of the plurality of different zones, wherein the composite score for a zone is indicative of either: (a) a disease or condition severity for a zone; or (b) a diagnosis for a zone; (iv) determine a lung score using a lung score algorithm analyzing the plurality of determined composite scores, wherein the lung score is indicative of either: (a) a disease or condition severity for the lung(s); or (b) a diagnosis for the lung(s); and

a user interface (240) configured to provide one or more of the lung score, the composite score for one or more of the plurality of different zones, and the confidence score for one or more of the identified one or more features.

10. The system of claim 9, wherein the temporal sequence of ultrasound image data comprises a plurality of frames for each of the plurality of zones, and further wherein a confidence score is determined based on an aggregation of a per-frame confidence score determined for each frame of the plurality of frames.

11. The system of claim 9, wherein the composite score algorithm determines a composite score for a zone by aggregating two or more composite scores for the zone.

12. The system of claim 9, wherein the processor is further configured to weight one or more composite scores, wherein the lung score algorithm utilizes the weighted composite score(s) when determining the lung score.

13. The system of claim 9, wherein the processor is further configured to receive, via the user interface, a request for additional information about a user-selected lung zone; and provide, via the user interface, the requested additional information about the user-selected lung zone, wherein the additional information comprises one or more of the temporal sequence of ultrasound image data for user-selected lung zone, the composite score for the user-selected lung zone, and the confidence score for the user-selected lung zone.

14. The system of claim 9, wherein the composite score for each of the plurality of different zones is determined by aggregating the identified features for the respective zone.

15. The system of claim 9, wherein the composite score for each of the plurality of different zones is determined by aggregating the same identified feature across the plurality of zones.

```
┌─────────────────────────────────────────────────────────────────┐
│                   Provide a lung analysis system                  │
│                               102                                 │
└─────────────────────────────────────────────────────────────────┘
```

100

┌─────────────────────────────────────────────────────────────────┐
│   Receive ultrasound image data for a plurality of zones of the lungs of a patient   │
│                               104                                 │
└─────────────────────────────────────────────────────────────────┘

┌─────────────────────────────────────────────────────────────────┐
│   Identify clinical features in each of the plurality of different zones   │
│                               106                                 │
└─────────────────────────────────────────────────────────────────┘

┌─────────────────────────────────────────────────────────────────┐
│   Determine confidence scores for the identified clinical features   │
│                               108                                 │
└─────────────────────────────────────────────────────────────────┘

┌───────────────────────────────┐        ┌───────────────────────────────┐
│   Determine a composite score for │  ──▷  │   Weight one or more of the determined │
│   each of the plurality of imaged │       │       composite scores        │
│              zones             │        │              111              │
│              110              │        │                               │
└───────────────────────────────┘        └───────────────────────────────┘

┌─────────────────────────────────────────────────────────────────┐
│   Determine an overall lung score using the plurality of composite scores   │
│                               112                                 │
└─────────────────────────────────────────────────────────────────┘

┌─────────────────────────────────────────────────────────────────┐
│   Provide the user with the lung score, composite scores, and/or confidence scores   │
│                               114                                 │
└─────────────────────────────────────────────────────────────────┘

┌─────────────────────────────────────────────────────────────────┐
│   Receive a request for additional information about a user-selected lung zone   │
│                               116                                 │
└─────────────────────────────────────────────────────────────────┘

┌─────────────────────────────────────────────────────────────────┐
│   Provide the requested additional information about the identified lung zone   │
│                               118                                 │
└─────────────────────────────────────────────────────────────────┘

FIG. 1

270

Ultrasound Device

200

220 230 240

Processor | Memory | User Interface

212

System Bus

261

Operating System

262

Ultrasound Imaging Data

263

Feature Identification Algorithm

264

Composite Score Algorithm

265

Lung Score Algorithm

266

Reporting Instructions

Communication Interface

250

Storage

260

FIG. 2

Severity scale (0 to 3) ⟶

300

| | 0 | 1 | 2 | 3 |
|---|---|---|---|---|
| ▸ Lung sliding | Yes | – | – | – |
| A-lines | Yes | – | – | – |
| B-lines | ≤2 B-lines | ≥3 B-lines | Merged (<50%) | Merged (≥50%) |
| Pleural line | Normal | Thick-ened | Irreg-ular | Inter-rupted |
| Consolidation | – | – | Small | Large |
| Pleural effusion | Alternative Dx | | | |

FIG. 3

Severity scale (0 to 3) ⟶

400

| | 0 | 1 | 2 | 3 |
|---|---|---|---|---|
| ▸ Lung sliding | Yes | – | – | – |
| A-lines | Yes | – | – | – |
| B-lines | ≤2 B-lines | ≥3 B-lines | Merged (<50%) | Merged (≥50%) |
| Pleural line | Normal | Thick-ened | Irreg-ular | Inter-rupted |
| Consolidation | – | – | Small | Large |
| Pleural effusion | Alternative Dx | | | |

*Clinical rules-based algorithm*
*for determining severity*

Overall score **8**

R1 0  L1 3  L3 1
R3 0
R2 0  L2 2
R4 0  L4 2

Overall severity score
for exam (all lung zones):

**0+0+0+0+3+2+1+2 = 8 points**

FIG. 4

FIG. 5

600

**For each LUS feature...**

Frame-level prediction/ detection per-feature

$Frame_1$ $Frame_2$ $Frame_3$ $Frame_4$ $Frame_5$ $Frame_K$

$size_1$ $size_3$ $size_4$ $size_5$ $size_K$

$+ \ldots +$

Video-level prediction per-feature

$$Pred_{video} = \frac{pred_1 + pred_2 + pred_3 + pred_4 + pred_5 + \cdots + pred_K}{K}$$

Approach 1 (average based on confidence scores)

$$Pred_{video} = \frac{size_1 + size_2 + size_3 + size_4 + size_5 + \cdots + size_K}{K}$$

Approach 2 (average based on detected feature size)

$$Pred_{video} = \sum_{i=1}^{K} (pred_i * size_i) \Big/ K$$

Approach 3 (average based on confidence score *and* feature size)

FIG. 6

18

*For each LUS feature...*

$Zone_1$ $Zone_2$ $Zone_3$ $Zone_4$ $Zone_N$

Video-level
prediction
per-feature

$pred_1$ $pred_2$ $pred_3$ $pred_4$ $+ ... +$ $pred_N$

Exam-level
prediction
per-feature

$$Pred_{Exam} = \frac{(pred_1 + pred_2 + pred_3 + pred_4 + ... + pred_N)}{N}$$ (approach 1)

FIG. 7A

*For each LUS feature...*

$Zone_1$ $Zone_2$ $Zone_3$ $Zone_4$ $Zone_N$

Frame-level
prediction
per-feature

$pred_K$
$pred_4$
$pred_3$
$pred_2$
$pred_1$

$+ ... +$

Exam-level
prediction
per-feature

$$Pred_{Exam} = \sum_{j=1}^{N}\sum_{i=1}^{K} pred_{ij} \Big/ (K * N)$$ (approach 2)

FIG. 7B

FIG. 8

Video-level predictions per-feature → Exam-level predictions per-feature → Overall diagnosis or severity prediction

**B-lines**

$Pred_{Zone1}$
$\vdots$
$Pred_{ZoneK}$

B-lines model

Single prediction for all lung zones

$Pred_{Exam}$

**Merged B-lines**

$Pred_{Zone1}$
$\vdots$
$Pred_{ZoneK}$

Merged B-lines model

Single prediction for all lung zones

$Pred_{Exam}$

**Pleural line abnormalities**

$Pred_{Zone1}$
$\vdots$
$Pred_{ZoneK}$

PL model

Single prediction for all lung zones

$Pred_{Exam}$

**Consolidation**

$Pred_{Zone1}$
$\vdots$
$Pred_{ZoneK}$

Consolidation model

Single prediction for all lung zones

$Pred_{Exam}$

**Pleural effusion**

$Pred_{Zone1}$
$\vdots$
$Pred_{ZoneK}$

Pleural effusion model

Single prediction for all lung zones

$Pred_{Exam}$

**Final Model (5 inputs)**

$Pred_{Final}$

FIG. 9

Video-level predictions per-feature

Overall diagnosis or severity prediction

**B-lines**
$Pred_{Zone1}$
$Pred_{Zone2}$
$\vdots$
$Pred_{ZoneK}$

Independent prediction per lung zone

**Merged B-lines**
$Pred_{Zone1}$
$Pred_{Zone2}$
$\vdots$
$Pred_{ZoneK}$

Independent prediction per lung zone

**Pleural line abnormalities**
$Pred_{Zone1}$
$Pred_{Zone2}$
$\vdots$
$Pred_{ZoneK}$

Independent prediction per lung zone

**Consolidation**
$Pred_{Zone1}$
$Pred_{Zone2}$
$\vdots$
$Pred_{ZoneK}$

Independent prediction per lung zone

**Pleural effusion**
$Pred_{Zone1}$
$Pred_{Zone2}$
$\vdots$
$Pred_{ZoneK}$

Independent prediction per lung zone

**Final Model (5*K inputs)**

$Pred_{Final}$

FIG. 10

Visual dashboard
summarizing overall
severity for the exam

Detailed results per-feature
for a particular lung zone

FIG. 11

Europäisches
Patentamt

European
Patent Office

Office européen
des brevets

# EUROPEAN SEARCH REPORT

**Application Number**

EP 22 15 0460

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 667 793 B2 (GEN ELECTRIC [US]) 2 June 2020 (2020-06-02) * col 6 lines 4-19; col 9 lines 30-55 * ----- | 1-15 | INV. G16H30/40 G16H50/20 |
| A | WO 2017/126753 A1 (SEOUL NAT UNIV BUNDANG HOSPITAL [KR]) 27 July 2017 (2017-07-27) * par 2, 35, 36, 40, 45 * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 21 June 2022 | Bankwitz, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

24

## ANNEX TO THE EUROPEAN SEARCH REPORT
## ON EUROPEAN PATENT APPLICATION NO.

EP 22 15 0460

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2022

| Patent document cited in search report | | Publication date | Patent family member(s) | | Publication date |
|---|---|---|---|---|---|
| US 10667793 | B2 | 02-06-2020 | NONE | | |
| WO 2017126753 | A1 | 27-07-2017 | KR 20170087714 | A | 31-07-2017 |
| | | | WO 2017126753 | A1 | 27-07-2017 |

For more details about this annex : see Official Journal of the European Patent Office, No. 12/82

EPO FORM P0459